# EUROPEAN PATENT SPECIFICATION

(11) **EP 1 360 329 B1**
(45) Date of publication and mention of the grant of the patent: **11.04.2007**
(21) Application number: 02711083.2
(22) Date of filing: 13.02.2002
(51) Int. Cl.: C12Q 1/68

(54) **BIOCHEMICAL METHOD AND APPARATUS FOR DETECTING GENETIC CHARACTERISTICS**
BIOCHEMISCHE METHODE UND APPARAT ZUR DETEKTION GENETISCHER CHARAKTERISTIKA
METHODE BIOCHIMIQUE ET APPAREIL ASSOCIE PERMETTANT DE DETECTER DES CARACTERISTIQUES GENETIQUES

(30) Priority: 13.02.2001 GB 0103464; 13.02.2001 GB 0103475; 13.02.2001 GB 0103471; 20.02.2001 GB 0104132
(43) Date of publication of application: 12.11.2003
(73) Proprietor: Pronostics Limited, Babraham Cambridge CB2 4AT (GB)
(72) Inventor: GAREY, Caroline, Cambridge CB4 1BN (GB); HADLEY, Jodie, Ely CB6 3WL (GB); ENGLAND, Mark, Cambridge CB4 6DG (GB)
(74) Representative: Sutcliffe, Nicholas Robert
(86) International application number: PCT/GB2002/000644
(87) International publication number: WO 2002/064829

(56) References cited:
- WO-A-00/55363
- WO-A-01/44812
- WO-A-95/32425
- WO-A-97/14028
- DE-A- 10 031 028
- US-A- 4 568 630
- US-A- 5 519 200
- US-A- 5 981 180
- US-A- 6 096 496

## Description

### Field of the Invention

This invention relates to a biochemical method of detecting genetic characteristics according to the preamble of appended Claim 1.

### Background to the Invention

During recent years, there has arisen a considerable interest in techniques and associated systems for determining genetic characteristics of numerous types of organisms, for example, yeast, bacteria and mammals. Earlier, tests for detecting genetic characteristics were performed manually in a sequential manner in laboratories. Later, technological developments relating to genetic characterisation evolved towards greater automation with associated higher detection throughput. Such technological developments have been prompted by, for example, the human genome project; this project has indicated that there are actually in the order of 30, 000 to 40, 000 genes in the human genome. With millions of genetic characteristics and thousands of different specimens to be analysed, high throughput methods of analysing genetic characteristics have become very important for the continued progress of genetic science. There is, for example, currently a need for massively parallel high throughput technologies for screening samples for gene expression and genotyping as well as for drug research and development. This need for high throughput methods has resulted in many new technologies and associated methods of determining genetic characteristics becoming commercially available.

There are several known techniques for determining genetic characteristics, these techniques involve a plurality of constituent experiments which are individually labelled; when the experiments have been completed, they can be read using their associated labels for identification. Labels used at present include:
(a) the position of each experiment on the surface of a test integrated circuit, known as a "DNA test chip";
(b) the position of each experiment in a microtitre plate or in a tube;
(c) the position of each experiment on the surface of a membrane; and
(d) fluorescent spectrum or other methods of identifying particles to which the experiments are bound.

Such known methods have the disadvantage of employing components for their execution which are difficult and expensive to manufacture and use. Moreover, the methods also suffer a high degree of background interference which limits their potential applications for genetic characterization purposes.

In a United States patent no. US 6, 027, 880, a microarray is described. The microarray concerns an integrated circuit whose surface is partitioned into a plurality of spatially disposed sites, each site corresponding to an individual experiment. Each individual experiment is.provided with one or more corresponding nucleotides thereat. Each site is effectively labelled by virtue of its spatial position on the surface of the integrated circuit. A company Affymetrix manufactures such microarrays, each microarray capable of analysing in the order of 12, 000 full-length genes by parallel analysis. As the number of samples tested on the same microarray has increased in recent years to several thousand, the demand for associated manufacturing equipment miniaturization and specialized materials handling has rendered the fabrication of such microarrays increasingly complex. The genetic characteristics of samples being monitored on such microarrays must often be known and isolated beforehand; such prior knowledge makes it a complicated and costly process to manufacture specific microarrays to customer requirements for each different type of organism or species to be studied. Further disadvantages associated with this technology are low flexibility, long manufacturing turnaround times, high cost and low data quality.

Initial investment costs for manufacturing the aforesaid microarrays are often considerable. A majority of potential users of such microarrays find their cost prohibitive. Moreover, there are high risks for potential users investing in equipment utilizing the microarrays, as the equipment is highly application specific and quickly become outdated. With few specialist buyers of this type of equipment, the resale value is often low.

Instrumentation apparatus, for example readers and robotic systems, used for performing microarray experiments are also technically advanced and hence very expensive, Further disadvantages are poor sensitivity and considerable background noise inhibiting precise determination of experimental results. Techniques have also been applied to improve the reaction kinetics and therefore the quality of results for microarrays. For example, improvements to surface-to-volume ratios of micro arrays through the use of channels and porous materials are described in Akzo Nobel's published international PCT application no. WO 99/02266. In practice, it has been found to be problematic to attain sufficient reaction kinetics when using such microarrays. Once again, these problems have resulted in complicated manufacture which has restricted the flexibility for the user to tailor experiments when using microarrays,

Bioassays conducted on micro-particles provide another type of massively parallel array technology and are presently in use. Methods of mutually separating different samples have been achieved by attaching information molecules to small supports so that many tests can be performed simultaneously. A system used to mutually distinguish the supports is normally fluorescence or reflection indexes.

In a published international PCT application no. WO 99/35293, there is described a method of analysing a genetic characteristic in the form of differential gene expression. The method includes a reference population of nucleic acid probes hybridised with reference DNA cloned on solid supports. The solid supports are microparticles and use optical labels to react with the polynucleotides to indicate an associated reaction. Advanced sorting apparatus is then used to sort out the supports that have reacted, such sorting achieved by way of differential optical signal intensity associated with the different supports; the optical labels are light emitting and the microparticles supports are sorted depending on the intensity of the light emitted therefrom. Such a sorting method allows greater flexibility than microarrays in the detection of genetic characteristics through the use of differently loaded microparticles. However, there are still problems experienced concerning the complexity of instrumentation required for determining the different intensity levels of light emitted from the activated microparticles.

WO-A-97/14028 and US-A-5,981,180 disclose a method for the multiplexed diagnostic and genetic analysis of enzymes, DNA fragments, antibodies, and other biomolecules. Flow cytometry is used to classify labelled beads within a beadset.

WO-A-00/55363 discloses a method of detecting and analysing differences between nucleic acids from two sources, wherein target beads are mixed with two nucleic acids and analysed by flow cytometry.

DE-A-10031028 discloses a method for selecting particles with a predetermined characteristic from a population comprising a large number of different particles.

WO-A-95/32425 discloses a method for encoding combinatorial libraries using fluorophore labeled beads, and US-A-6,096,496 discloses a combinatorial chemistry bead that includes an electromagnetic spectral emitter as a unique identifier.

US-A-4,568,630 discloses a method of preparing an anodized aluminum printing plate.

US-A-5,519,200 discloses an identification device including a magneto-optical strip and an optical bar code, wherein the magneto-optical strip is positioned in a known location relative to the optical bar code such that on visual identification of the bar code the location of the magneto-optical strip can be found.

In the Applicant's published international PCT application no. WO 00/16893, there is described an innovation concerning the use of solid supports in a bioassay, and a process for manufacturing such supports. The supports are fabricated from an anodised metal, preferably aluminium. The supports have, for example, antibodies attached thereto for bonding to antigens.

Contemporary methods of detecting genetic characteristics concern gene expression profiling and genotyping analysis. Such analyses are currently executed using DNA chips or microarrays and spot arrays. These methods have the disadvantage of variable quality of spotting, which may result in low reliability of test results thereby obtained from the arrays. Such low reliability has, in turn, resulted in extensive quality control requirements during manufacture of the microarrays and spot arrays to ensure the quality of spotting. Moreover, the reproducibility of hybridisation has proved to be difficult to ensure during manufacture; difficulty in ensuring reproducible hybridisation has lead to difficulties in attaining reliable results when reproducing experimental results.

More recently, colour-coded microspheres have been used for genotyping and gene expression experiments. These experiments are however limited in their number of codes, relatively high cost of manufacture and therefore restricted regarding the number of tests that can be performed at any one time. Further disadvantages with this technology are the high cost of instrumentation required to read experiment results, and unfavourable absorption and emission properties of dyes used.

Another known approach for detecting genetic characteristics is Single Nucleotide Polymorphism (SNP) detection and scoring methods. There are many methods of SNP detection and scoring which exhibit various drawbacks. Some of the methods included in such SNP detection include miniature hybridisation array (DNA-chip), gel-based analysis and dynamic allele-specific hybridisation (DASH). These methods may also be used when detecting genetic characteristics for drug target association and pharmacogenomics. The methods have the disadvantage of requiring target PCR amplification; such amplification represents a burden that limits possibilities for scale-up and automation. Most other disadvantages mentioned for the aforesaid microarrays and bioassays, for example variable quality of spotting, reproducibility of hybridisation, and the limited number of samples that can be run at any instance, also apply to these methods.

Another problem experienced with contemporary genetic characterization technology is the need for staff to be highly trained and to understand several different system set-ups required when performing increasing numbers of experiments for determining genetic characteristics. Such staff requirements result in relatively large initial investments in staff training. It is often necessary, on account of validation requirements and to increase reliability of analysis results, to run experiments repetitively requiring supervision by scientists, which reduces the availability of these scientists for other activities. Moreover, in industries such as drug research and development, there are wide ranges of technologies used throughout the process that must all be validated resulting in considerable time, requirements and costs.

### Summary of the Invention

A first object of the invention is to provide an improved method of detecting genetic characteristics.

A second object of the invention is to provide a low cost high-throughput method of performing experiments for detecting genetic characteristics.

According to the invention, in order to address one or more of the aforesaid objects of the invention and other objects that will appear from the following specification, there is provided a method as defined in the accompanying Claim 1.

The method is of advantage in that it is capable of addressing the aforesaid objects of the invention.

Thus, the present invention concerns a method for detecting genetic characteristics, where supports with specific bar codes have an information molecule attached to a main surface thereof. Attaching the molecules onto the supports and suspending them in a fluid allows for very good reaction kinetics, thereby improving sensitivity as well as reducing the reaction volume and time. The sample potentially containing one or more genetic characteristics being detected is added to the fluid. A multiplexed experiment of hundreds of thousands of tests in one is possible since a large number of supports with different bar codes and attached information molecules can be present in the bioassay simultaneously. Use of such molecules in combination with supports decreases the need to perform batched or repeated experiments. Different types of signals are used to indicate the bar codes of the supports and the interaction signal indicating interaction with one or more genetic characteristics. Such an approach results in less advanced reader and detector units being required for performing assay measurements, thereby potentially reducing cost.

In a preferred embodiment of the invention, the supports are oxidised prior to the attachment of information molecules thereto. Such attachment allows the surface of the supports to have improved mechanical and chemical attachment properties, Alternatively, or additionally, the supports are coated in one or more molecular binding agents to enhance information molecule attachment thereto.

In a further preferred embodiment of the invention, a measuring unit performs the detection of signal emitting labels and the reading of the bar code substantially simultaneously. This simultaneous measurement decreases the risk of incorrect readings and increases the throughput as advanced software is not employed for the tracking of the supports.

In an additional embodiment of the invention, the reading of the bar code includes locating one or more features arranged to indicate how to interpret the information gathered. This makes it possible to identify the supports irrespectively of their position or flow direction through, for example, a flow cytometer reader system.

A further embodiment of the invention has the fluid including loaded supports placed on and subsequently affixed onto a substrate. This allows a multiple increase of the throughput capacity of the standard planar reading methods while only requiring minor adjustments to existing equipment set-ups.

According to a special approach of the invention, the measuring unit's reading involves conveying the substrate with its associated supports along a predetermined path. Such motion along the path is preferably achieved by moving the substrate with supports located thereon while the measuring unit is stationary. It is apparent that, alternatively, the measuring unit could be moved while the substrate with supports is stationary. Such approaches are capable of resulting in substantially all supports in the fluid being analysed. Those supports that are only partially in the measuring unit's focal area along the measuring path have their corresponding positions registered so that they are only analysed once.

In other preferred embodiments of the invention, the genetic characteristics detected are for gene expression, SNPs analysis/scoring, nucleic acid testing, drug target association or pharmacogenomics. These embodiments of the invention include a system for carrying out massively parallel multiple bioassay tests for gene expression analysis, SNPs analysis/scoring, drug target association, pharmacogenomics and/or nucleic acid testing in a low-cost, fast and convenient manner. Such a scheme achieves high throughput by making a suspension including many thousands of different types of, for example, micromachined coded supports, also called labels or micro-labels. Each of these supports carries nucleic acid or peptide nucleic acid (PNA) information molecules. The supports with attached information molecules are mixed with the sample potentially including the genetic characteristic under test together with a signal emitting label, namely a reporter system such as fluorescence. Only supports with nucleic acid probes or PNAs that bind to the genetic characteristics investigated will bind to the signal emitting label which then emits a signal, for example fluoresce.

An apparatus for detecting genetic characteristics may have detecting means and identifying means arranged to register two different types of signals, the first signal being associated with the detection of activated signal emitting labels and the second signal being associated with the reading of bar codes of supports. Such plurality of different types of signal decreases the potential requirement of using advanced and costly image processing equipment.

An embodiment of a solid support suitably used with such an apparatus in a gene expression, SNPs detecting/scoring, drug target association, or pharmacogenomic biochemical assay, is substantially linear or planar in shape and has an anodised metal surface layer. The largest dimension of the support is preferably less than circa 250 µm, more preferably less than 150 µm, and most preferably less than circa 100 µm in length, whereby an aqueous suspension is formable from a plurality of the supports. This allows the same type of bioassay to be used for several different experiment types.

In further embodiments, the support's surface layer has a cellular-structure anodisation layer with the growth direction of the cells of the anodisation layer being perpendicular to the plane of the surface layer. Suitably the support has nucleic acid or PNA information molecules (probe) bound to the surface layer. The support's surface layer may be of aluminium and may also be porous. Furthermore the pore size of the surface layer is suitably approximately matched to the size of the nucleic acid or PNA molecules to be bound. This provides the support with excellent mechanical and chemical bonding properties for the attachment of information molecules.

The bar code incorporated in the support is a spatially varying pattern for identification purposes. Suitably a measuring unit, for example an optical reader, is used for reading the patterns and identifying the supports.

It will be appreciated that features of the invention described in the foregoing can be combined in any combination without departing from the scope of the invention.

### Description of the Drawings

Embodiments of the invention will now be described, by way of example only, with reference to the accompanying drawings wherein:
- Figure 1: is a plan view and a side view of a single support comprising a bar code;
- Figure 2: is a schematic diagram of a bioassay comprising supports, information molecules and signal emitting labels;
- Figure 3: is a cross sectional view in the flow direction of a flow-based reader;
- Figure 4: is a schematic flow diagram of the incubation and reading process of a planar-based reader;
- Figure 5: is a schematic diagram illustrating a planar-based reader for interrogating supports on a planar substrate; and
- Figures 6a, 6b: are schematic top views of a planar substrate illustrating examples of the measuring path talcen by the planar-based reader.

### Description of Embodiments of the Invention

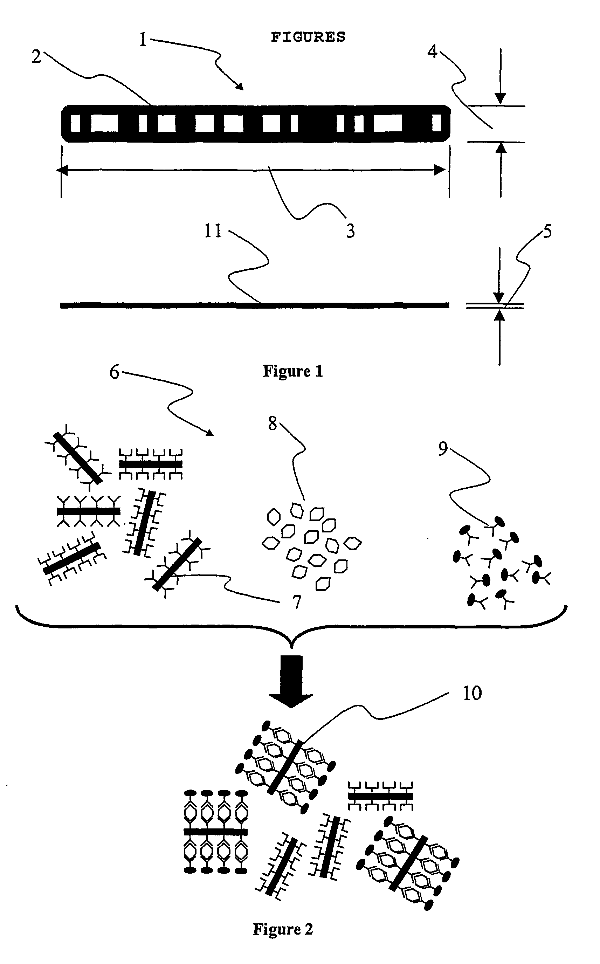

In Figure 1, an illustration of a preferred single support 1 is provided. Such a support will also be referred to as a "micro label" in the following description. The support 1 can be fabricated from a wide variety of materials ranging from polymers, glasses to metal alloys, but is preferably fabricated from a metal and most preferably fabricated from aluminium. The support 1 incorporates a bar code 2 which can be in the shape of at least one (or any combination thereof) of grooves, notches, depressions, protrusions, projections, and most preferably holes. The bar code 2 is suitably a transmission optical bar-code. The bar code 2 is implemented as a spatially sequential series of holes extending through the support 1. Such holes can be of varied shape and size. They are also capable of providing a very good optical contrast as solid areas of the support 1 are substantially non-transmissive to light whereas holes of the bar code 2 are highly transmissive to light received thereat.

The support 1 can be of many different types of shape, but has preferably a substantially planar form with at least a principal surface 11. Each support 1 of this type has a largest dimension 3 of less than circa 250 µm, more preferably less than 150 µm, and most preferably less than circa 100 µm in length. The support 1 has suitably a width 4 to length 3 ratio in a range of circa 1:2 to circa 1:20, although a ratio range of circa 1:5 to circa 1:15 is especially preferred. Moreover, the support 1 has a thickness 5 which is preferably less than circa 3 µm; and more preferably less than circa 1µm. The thickness of less than circa 1 µm has been shown to provide sufficient mechanical support strength for rendering the support 1 useable in bioassays. A preferred embodiment of the invention concerns supports 1 having a length 3 of circa 100 µm, a width 4 of circa 10 µm and a thickness 5 of circa 1 µm; such supports are capable of storing up to 100, 000 different identification sequence bar codes 2. Experimental demonstrations of up to 100, 000 different variants of the supports 1, for use in bioassays for genetic characterization experiments have been undertaken. Supports 1 of different lengths 3 in a range of 40 to 100 µm, carrying between two and five decimal digits of data in the bar code 2, have been fabricated for use in different experiments for the detection of genetic characteristics.

Around ten million such supports 1, namely micro-labels, can be fabricated on a single 6-inch diameter substrate, for example a silicon wafer, using contemporary established manufacturing techniques. Conventional photolithography and dry etching processes are examples of such manufacturing techniques used to manufacture and pattern an anodised aluminium layer to yield separate solid supports 1.

A fabrication process for manufacturing a plurality of supports similar to the support 1 involves the following steps:
(1) depositing a soluble release layer onto a planar substrate;
(2) depositing a layer of aluminium material onto the release layer remote from the substrate;
(3) defining support features in the aluminium material layer by way of photolithographic processes and etching processes;
(4) optionally anodising the aluminium material layer; and
(5) removing the release layer using an appropriate solvent to yield the supports released from the planar substrate.

It will be appreciated that steps (3) and (4) can be executed in either order. Moreover, if required, step (4) can be omitted. Optionally, gaseous anodisation of the aluminium material during step (2) can be employed; such gaseous anodisation is capable of imparting to the supports 1 anodisation regions extending deeply into the supports 1. The release layer is preferably polymethyl methacrylate (PMMA) or other suitable type of material, for example an optical resist as employed in conventional semiconductor microfabrication; the release layer is selected to exhibit properties allowing the aluminium material layer to be held in place with respect to the planar substrate during steps (3) and (4). When PMMA is employed, a suitable solvent comprises acetone and/or methyl isobutyl ketone (MIBK).

Referring now to Figure 2, there is shown a method of detecting genetic characteristics in the form of a bioassay indicated generally by 6. The bioassay 6 comprises two binding event experiments denoted by mutually different exposed molecular groupings as illustrated. The assay 6 comprises a plurality of supports, each support being similar to the support 1. Moreover, the assay 6 is generated by mixing together suspensions of chosen sets of active supports 1. Each active support 1 with a corresponding specific bar code 2 has associated therewith a unique information molecule 7, for example a nucleic acid or PNA probe associated therewith, which binds to and/or interacts with a specific type of sample molecule 8 detected during subsequent genetic characterization analysis. Information molecules 7 are used in a generic meaning rather then being limited to the meaning of a molecule in its physical or chemical meaning. The information molecules 7 may be attached to the supports 1 either before or after the supports 1 are released from a corresponding planar substrate employed during their fabrication. Enhanced coating of the information molecules 7 onto the supports 1 is achieved by attaching the molecules 7 to the supports 1 after their release from their associated planar manufacturing substrate. Signal emitting labels, for example a label 9, are preferably fluorescent labels. Only supports with information molecules 7 that have bound to the genetic characteristic sample molecule 8 detected will fluoresce. The fluorescent label 9 that is bound to the sample molecule 8 detected and indirectly the information molecule 7 causes this fluorescence, denoted by 10. The sample molecule 8 preferably comprises matter for genetic characteristic detection. The sample molecule 8 is preferably labelled with the signal emitting labels 9 before being introduced into the bioassay 6, namely a fluid, preferably a liquid solution and most preferably a liquid solution including water. Alternatively, the signal emitting labels 9 can be introduced into the liquid solution prior to adding the sample to be genetically characterised The result of the test is measured by the degree of fluorescence of different types of supports 1. The fluorescent intensity of the signal emitting labels 9 quantifies the level of detected sample molecules 8 with the genetic characteristics present in the bioassay 6. Experiments where a binary yes/no reaction indication is preferred only require determination whether or not the supports 1 in the bioassay 6 are fluorescent.

The information molecules 7 attached to the supports 1 are preferably used in experiments for detecting sample molecules with specific genetic characteristics in different embodiments of the invention, for example the molecules 7 can be:
(a) nucleic acid and/or PNA molecules for gene expression analysis;
(b) nucleic acid and/or PNA molecules for Single Nucleotide Polymorphisms (SNP) analysis;
(c) nucleic acid and/or PNA molecules for nucleic acid testing;
(d) nucleic acid and/or PNA molecules for drug target association; or
(e) nucleic acid and/or PNA molecules for pharmacogenomics.

It will be appreciated that the information molecules 7 are not limited to (a) to (e) above and can comprise a broad range of compounds capable of being uniquely distinguished and identified An example of a suitable compound is a DNA binding protein and more preferably a single strand binding protein. All molecules in this broad range and/or probes may be attached to supports fabricated by steps (1) to (5) above either before or after executing photolithographic operations or releasing the supports 1 from the planar substrate. The information molecules 7 are preferably attached only to one side of the support 1; alternatively, the molecules 7 preferably cover the support 1 in whole or partially.

The molecules 7 can be arranged to bind only wealdy to the supports 1; such weak binding is achieved by arranging for the aluminium surface 1 to be in an untreated state when incubated in a liquid solution, for example an aqueous solution. By modifying the surface 11 of the supports 1 or the information molecules 7, such binding can be selectively enhanced. Anodising the attachment surface 11 of the supports 1 is one way of providing such enhancement. Methods of growing porous surfaces on aluminium are known in the art. Likewise, processes for sealing such porous surfaces are also known. The Applicant has exploited such knowledge to develop a relatively simple process for growing an absorbing surface having accurately controlled porosity and depth. Such porous surfaces are capable of binding well to preferred nucleic acid or PNA molecules. Using an avidin-biotin system is another approach for improving binding between the supports 1 and their associated information molecules 7. The support's 1 surface 1 may also be treated with a polymer material such as silane and/or biotin, to further enhance attachment properties. The supports 1 preferably have silane baked onto their surfaces 11. Attaching linking molecules, for example avidin-biotin sandwich system, to the information molecules 7 further enhances their chemical molecular attachment properties.

Such enhanced attachment is important because it allows the probe molecules 7 to be bound strongly to the support surface 11 during manufacture whilst maintaining weak non-specific binding of fluorescent target molecules 8 during tests. Moreover, such enhanced attachment is preferably achieved through having covalent bonds between attachment surface 11 of the support 1 and the information molecule 7. The covalent bonds prevent the information molecules 7 from being dislodged from the supports 1 and causing disturbing background noise in the bioassay 6 during analysis. It is found to be important to wash the active supports 1, said supports having information molecules 7 attached thereto, after attachment to remove any excess information molecules 7 that could otherwise increase the noise in the bioassay 6 during analysis. Discrimination of the tests is thereby enhanced through a better signal-to-noise ratio.

As described in foregoing, each different bar code 2 fabricated onto the supports 1 is associated with a unique corresponding information molecule 7. The bar code 2 is preferably stored on the supports 1 as a series of holes using coding schemes similar to those found on conventional bar code systems, for example as employed for labelling merchandise in commercial retailing outlets. Such a code allows the use of existing reader technology to identify the bar-codes 2 of the supports 1, thereby decreasing the initial investment when adopting technology according to the invention.

Reader systems for use with the bioassay 6 and associated supports will now be described.

The Applicant has developed two classes of reader system. These are based on flow cells for handling the supports 1, and on planar imaging of plated-out supports 1.

A flow-based reader system, similar in construction to a flow cytometer, can be used to draw through thousands of supports 1 per second, thereby reading simultaneously the bar code 2 of each support 1 and the results of its associated test result. The test result is measured as a yes/no binary result or by the degree of fluorescence 10. Alternatively, a planar reader system can be employed, wherein:
(a) the supports 1 are plated out onto a planar substrate; and then
(b) fluorescence microscopy and associated image processing are employed to read the bar codes of the supports and the results of their associated tests,

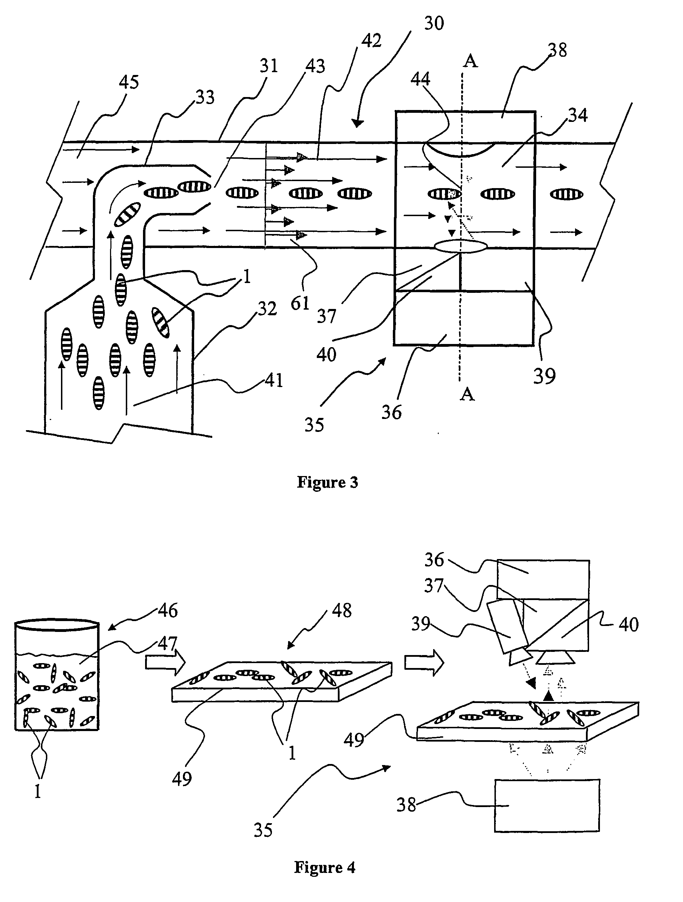

Embodiments of the flow-based reader system and the planar reader system will now be described in further detail with reference to Figures 3, 4, 5 and 6.

Referring to Figure 3, there is shown a flow-cell reader indicated generally by 30. The reader 30 comprises a flow tube 31 having an upstream end and a downstream end. At the upstream end, there is included within the tube 31 an injection nozzle 33 in fluid communication with an associated focussing zone 32, the zone 32 being situated outside the tube 31. The zone 32 is tapered where it interfaces to the nozzle 33. Moreover, the nozzle 33 comprises at its remote end within the tube 31 an exit aperture 43.

At the downstream end, the reader 30 comprises a measuring unit indicated by 35 for reading supports 1 conveyed in operation in fluid flow from the nozzle 33 at the upstream end to the measuring apparatus 35 at the downstream end. The apparatus 35 includes a reading zone 34, a reader unit 37, a light source 38, a detector unit 40, a signal emitting unit 39 and a processing unit 36. The signal emitting unit 39 is preferably a fluorescent source.

Operation of the reader 30 will be described initially in overview.

A bioassay 6, for example a liquid comprising a plurality of the supports 1 dispersed therein, is introduced into the focussing zone 32. Moreover, a flow of fluid 45, for example filtered water, is generated along the tube 31 in a direction from the upstream end towards the downstream end. Supports 1 in the focussing zone 32 are encouraged, by the tapered profile of the zone 32, to align into a row-like formation as illustrated. The supports 1 are ejected from the exit aperture 43 and are swept in the flow 45 along the tube 31 into the reading zone 34 and eventually therepast. When one or more of the supports 1 enter the reading zone 34, light from the source 38 illuminates the one or more supports 1 so that they appear in silhouette view at the reader unit 37. The reader unit 37 generates a corresponding silhouette signal which is communicated to the processing unit 36 for subsequent image processing to determine the bar code 2 of the supports 1. The signal emitting unit 39 also illuminates the zone 34 with radiation having a wavelength selected to induce fluorescence in one or more the active supports 1. The detector unit 39 detects any fluorescence occurring in the zone 34 and generates a corresponding fluorescence signal which is subsequently received by the processing unit 36. For each support 1 transported through the zone 34, the processing unit 36 is programmed to determine the bar code 2 of the support 1 with its corresponding magnitude of fluorescence. Moreover, the processing unit 36 is also connected to an associated data base relating the bar code 2 with a test provided by its associated information molecules 7.

Preferably, the fluid 45 flowing in operation along the tube 31 is a liquid. Alternatively, the fluid 45 can be a gas at reduced pressure relative to the nozzle 33 so that liquid bearing the supports 1 to the exit aperture 43 is vapored at the aperture 43, thereby assisting to launch supports 1 into the tube 31. Whereas it is easier to establish a laminar flow regime within the tube 31 when fluid flowing therethrough is a liquid, gas flow through the tube 31 potentially offers extremely fast support 1 throughput and associated interrogation in the zone 34.

Design and operation of the reader 30 will now be described in more detail.

The reader 30 is designed to induce the supports 1, namely micro-labels, to flow along a central region of a tube 31 through the defined interrogation zone 34. By utilizing an accelerated sheath fluid 41 configuration and the injecting nozzle 33, the supports 1 injected into the central region of the tube 31 are subjected to a hydrodynamic focusing effect 42 causing all the supports 1 to align lengthwise, namely axially, and to pass through a well-defined focal point 44 in the interrogation zone 34 downstream from an exit aperture 43. In the tube 31, there is a laminar flow of a reading fluid 45 which mixes with the bioassay solution 6 entering the tube 31 through the injection nozzle 33. The distance between the exit aperture 43 and the interrogation zone 34 must be sufficiently long to dissipate any turbulence caused by the injection nozzle 33. This sufficient length allows for a substantially laminar flow of the reading fluid 45 and hence provides the supports 1 with a non-oscillating movement past the focal point 44. If required, the nozzle 33 can be provided with a radially symmetrical arrangement of feed tubelets from the focussing zone 32 so as to obtain a more symmetrical velocity profile within the tube 31. A velocity profile 61 included in Figure 3 provides an illustration of the velocity of the substantially laminar fluid flow in the tube 31; fluid velocity increases from a central region of the tube 31 towards interior peripheral surfaces of the tube 31. In an interface surface region in close proximity to the peripheral surfaces of the tube 31, fluid velocity progressively reduces to substantially zero at the interior surface of the tube 31.

Prior to entering the tube 31, the supports 1 pass through the focusing zone 32 which is operable to arrange the supports 1 for injection into the tube 31. The supports 1 are transported through the tube 31 to the interrogation zone 34 where they are interrogated by the measuring unit 35 when at the focal point 44. Preferably, the supports 1 used in the flow-based reader system 30 have information molecules 7 attached on at least two opposite principal surfaces 11 of the supports 1.

The light source 38 emits light that passes though the reading zone 34 and illuminates the support 1 at the focal point 44. Preferably, the light source 38 emits light in a plane A-A. that is substantially perpendicular to the bioassay's flow 45 direction and from two different radial directions, the radial directions preferably having a mutual angle separation, for example with a mutual angular separation of circa 45° separation. Such an arrangement of support 1 illumination in the focal point 44 enables the supports 1 to be identified irrespectively of their rotational position along their longitudinal axis. The reader unit 37, located substantially at an opposite side of the interrogation zone 34 relative to the light source 38, reads the light that passes through one or more supports 1 at the focal point 44, The reader unit 37 is in optical communication with the supports when they pass through the interrogation zone 34. A feature in the form of a marking at one end of each support 1 is used to indicate to the reader unit 37 how to interpret the read information. This allows the support 1 to be read from either direction along its longitudinal axis. The marking is also susceptible to being used to increase the number of possible bar codes on a support 1 to be greatly in excess of 100,000. For example, employing four different markings on separate sets of supports 1 is capable of increasing the number of identification combinations of supports to about 400, 000. An alternative feature to indicate how information codes are to be read is to start each block with 0's and end the blocks with 1's, or vice versa. Further alternatives of these features preferably error checking data, for parity bit checks and/or forward error correction, thereby improving testing reliability.

In operation, the signal emitting unit 39 emits radiation, for example fluorescent light, that causes the supports 1 that have reacted with the sample molecules 8 and the signal emitting label 9 to give off corresponding fluorescent radiation 10. The detector unit 40 measures the magnitude of the intensity of the fluorescent radiation 10 that is given off by the activated signal labels 9 on the supports 1. This intensity indicates the degree of reaction which can be extrapolated to determine the amount of reactive sample molecule 8 present in the genetic characteristic bioassay 6 sample. The processing unit 36 then evaluates the information from the detected bar codes 2 of the supports 1 measured by the reader unit 37 and to what extent those supports 1 have given off a signal 10 detected by the detector unit 40. The information is then verified with corresponding information in a database comprising preset information linking specific bar codes 2 to specific information molecules 7.

Once a sufficient number of supports 1 have been read, the processing unit 36 of the measuring unit 35 calculates the results of the tests associated with the supports 1. This sufficient number is preferably between 10 and 100 copies of each type of supports 1, this number is preferably to enable statistical analysis to be performed on test results. For example, statistical analysis such as mean calculation and standard deviation calculation can be executed for fluorescence 10 associated with each type of information molecule 8 present The processing unit 36 also controls the reader and detector units 37, 40 so that the each individual support 1 is only analysed once. It could also be possible to only analyse the fluorescent 10 supports 1 that pass through the flow reader 30 to lower the amount of information processed.

In Figure 4, there are shown an incubation process 46 comprising the steps of :
(a) placing supports 1 on a planar substrate 49, for example a chip, glass slide or microarray, to provide a corresponding support-loaded substrate 48, and
(b) interrogating the support-loaded substrate 48 using a planar measuring unit 35 as illustrated in Figure 3 and described in the foregoing.

The incubation process 46 involves mixing supports 1 bearing attached information molecules 7 with a sample comprising genetic characteristic molecules 8 in a liquid bioassay solution 6. The supports 1 are then deposited on the planar substrate 49 and can be subsequently dried to generate the support-loaded substrate 48. Next, the measuring unit 35 measures the level of fluorescence 10 and also the bar codes 2 of the different supports 1 of the support-loaded substrate 48. Normally, all the supports I on the loaded substrate 48 are analysed to verify the total quality of the experiment. In cases where there could be an interest in saving time and/or processing capacity, the software of the processing unit 36 can preferably be configured to analyse only the supports 1 that give off a signal 10, for example through a fluorescent signal label 9, indicating that an interaction with the genetic characteristic molecules 8 has occurred. The analysis of the loaded substrate 48 using the planar measuring unit 35 is a very cost effective, easy to perform and suitable way to multiply the analysing capacity for low to medium sample numbers in the range of, for example, single figures to a few thousand supports on each substrate 48.

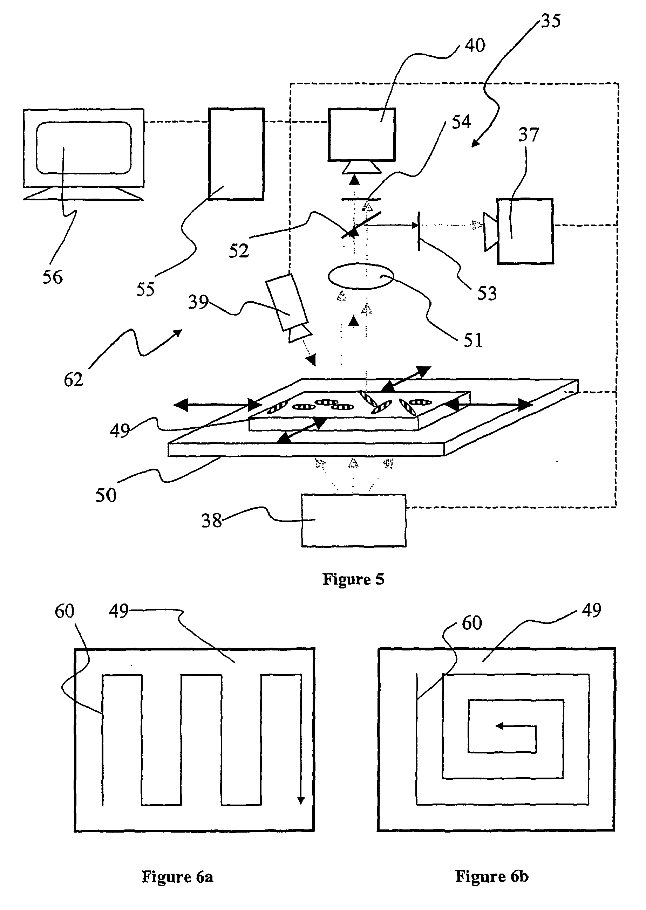

A planar reader system is illustrated in Figure 5 and indicated generally by 62. In the reader 62, supports 1 are plated out, namely fixedly deposited or deposited in a liquid, onto the planar light-transmissive substrate 49. Preferably, the planar substrate 49 is fabricated from a polymer, glass or silicon-based material, for example a microscope slide, and most preferably it is in the form of a microarray. Thereafter, the measuring unit 35 arranged to perform conventional fluorescence microscopy is used to analyse the support-plated substrate 49 systematically. Preferred paths 60 for systematically interrogating the substrate 49 are shown in Figure 6a and 6b. Figure 6a is a depiction of a meander-type interrogation regime, whereas Figure 6b is a depiction of a spiral-type interrogation regime. There are of course many other possible paths 60 apparent to one skilled in the art, for example moving the substrate 49 in an opposite direction to the path 60, or moving the substrate in a meandering diagonal path. However, the regimes of Figures 6a, 6b are efficient for achieving an enhanced support 1 read speed. Preferably, a stepper-motor actuated base plate 50 supporting and bearing the substrate 49 is used to move the substrate 49 around while the measuring unit 35 is held stationary. The positions of supports 1 are tracked so that they are analysed once only.

The planar measuring unit's 35 reader unit 37 for image-processing is used to capture digital images of each field of the substrate 49 to which supports 1 have become affixed. Digital images thereby obtained correspond to light transmitted through the substrate 49 and base plate 50 and then through the supports 1 rendering the supports 1 in silhouette view; such silhouette images of the supports 1 are analysed by the reader unit 37 in combination with a processing unit 55. The bar code 2 associated with each support 1 is hence identified from its transmitted light profile by the reader unit 37. The signal emitting unit 39 generates a fluorescent signal, which signal makes the labels 9 on supports 1 that have interacted with the genetic characteristic molecules 8 fluoresce 10. A detector unit 40 detects the magnitude of fluorescence 10 from activated supports 1 to identify the degree of reaction. The fluorescent signal 10 integrated over activated supports' 1 surface 11 is recorded in association with the identification bar-code 2 to construct data sets susceptible to statistical analysis.

The processing unit 55 is connected to the light source 38, the signal unit 39, the reader unit 37, and the detector unit 40 and to a display 56. Moreover, the processing unit 55 comprises a control system for controlling the light source 38 and the signal unit 39. The light silhouette and fluorescent signals 10 from the supports 1 pass via an optical assembly 51, for example an assembly comprising one or more lenses and/or one or more mirrors, towards the detector unit 40 and reader unit 37. A mirror 52 is used to divide the optical signals into two paths and optical filters 53, 54 are used to filter out unwanted optical signals based on their wavelength. Alternatively, the light source 38 and signal unit 39 can be turned on and off at intervals, for example mutually alternately. Signals are received from the reader unit 37 and detector unit 40, which are processed and corresponding statistical analysis results presented on a display 56. Similar numbers of each type of supports 1 are required to give optimal statistical analysis of experiments. Such statistical analysis is well known in the art.

The preferred embodiment of the biochemical method of detecting one or more genetic characteristics utilises the supports 1 with bar code 2 described previously. The method comprises several steps, which can be performed in several different orders, and will now be described in more detail.

Information molecules 7 are attached to at least a main surface 11 of the supports 1 to allow the detection of potential genetic characteristic matter 8 in a sample. Supports 1 with at least one type of bar code 2 are then suspended in a fluid 6 to allow a 3-dimensional array where the supports 1 are submersed in the fluid 6. The 3-dimensional array allows for very good reaction kinetics. The number of different types of supports I suspended in the fluid 6 is dependant on the test throughput required, but could be hundreds, thousands or even millions. The number of the same types of supports 1 suspended in the fluid 6 is amongst other things dependent on quality of statistical analysis and the ease of analysis.

The sample, potentially containing genetic characteristic matter 8, to be analysed is added to the fluid 6 before or after the supports 1 have been suspended in the fluid. Signal emitting labels 9 are also added to the fluid 6. These signal emitting labels 9 are used to indicate interaction, e.g. bonding, between the information molecules 7 on the supports 1 and the genetic characteristic matter 8 sought in the analysed sample. There are many different ways of adding the signal emitting labels 9 to the fluid 6. They can, for example, be added to the fluid 6 separately, be attached to the genetic characteristic matter 8 to be analysed prior to the sample being added to the fluid 6, or be attached to the information molecule 7 before or after their attachment to the supports 1. There are also many different ways for the signal emitting labels 9 to indicate that interaction between the information molecules 7 and the genetic characteristic matter 8 in the analysed sample,

One such way is for a signal, such as fluorescence or light of other wavelength (colour), to be activated by the signal emitting label 9 if there is interaction between an information molecule 7, a matching genetic characteristic matter 8 and the signal emitting label 9. Alternatively the signal emitting labels 9 are activated before any interaction with the genetic characteristic matter 8. When there is an interaction between the information molecule 7 and the genetic characteristic matter 8 the active signal emitting label 9 is released from the other molecules deactivating its signal. This would result in a detection that is opposite to the ones discussed previously, i.e. the absence of a signal indicates that a reaction has occurred on a support in e.g. a yes/no experiment Similarly a decrease in the fluorescent signal 10 can be an indicator of the amount of genetic characteristic matter 8 present in the analysed sample introduced into the fluid 6.

The fluid 6 containing supports 1 with information molecules 7, the sample to be analysed 8 and the signal emitting labels 9 is analysed using a detecting unit 40 and a reader unit 37. The reader unit 37 reads the bar codes 2 of at least those supports 1 with information molecules 7 that have reacted with the genetic characteristic matter 8 in the analysed sample. It may also be preferred to read the bar codes 2 of all the supports 1 as a quality control of the multiplexed experiment. The detection unit 40 detects the absence or presence of interaction signals 10 of the signal emitting labels 9. In an alternative type of biochemical assay method more than one signal may be used on each support indicating the presence of two or more genetic characteristics 8 in the analysed sample. This would mean that two or more different information molecules 7 were attached to the same support 1. In such a case the signal emitting labels 9 would give off a different signal 10 depending on the genetic characteristic matter 8 bonding to the information molecules 7. Another preferred methodology used for the detection of genetic characteristics, such as different genotypes, is to use the combined signal from two or more supports 1 with different bar codes 2 to indicate the presence of the genetic characteristic. The signal combinations could, for example, be an active support A and passive support B, active supports A and B, or a passive support B and active support A, each different combination of supports indicating what type of genetic characteristic is detected in the fluid.

The intended uses of the biochemical assay for detecting one or more genetic characteristic include gene expression, SNPs analysis and nucleic acid testing. These uses of the bioassay methods are suitable for use in the field of drug target association, pharmacogenomics and diagnostics.

It will be appreciated that modifications can be made to embodiments of the invention described in the foregoing without departing from the scope of the invention as defined by the appended claims.

## Claims

1. A biochemical method of detecting one or more genetic characteristics, the method utilizing supports (1) whose largest dimension (3) is less than 250 µm and wherein each support (1) incorporates sequential identification means (2), the method comprising the steps of:
(a) attaching an information molecule (7) to a main surface (11) of each support (1);
(b) suspending the supports (1) with associated information molecule (7) in a fluid;
(c) adding a sample (8) to be analysed to the fluid;
(d) placing the fluid comprising its associated supports (1) and sample (8) on a substrate (49) for subsequent interrogation;
(e) detecting interaction signals from at least one of the supports (1) in the fluid using signal detecting means (40); and
(f) reading the sequential identification means (2) of the supports (1) which have an interaction signal using reading means (3), thereby detecting at least one of said one or more genetic characteristics (8),
**characterised in that**
(g) the information molecule (7) is capable of interacting with at least one of said one or more genetic characteristics; and
(h) the sequential identification means (2) is a bar code and integrally incorporates at least one of spatial orientation features and error correction features for assisting the reading means (3) to determine identities of the supports (1).

2. A method according to Claim 1, **characterised in that** the method further includes a step of oxidizing the supports (1) prior to attachment of associated information molecules (7) thereto.

3. A method according to Claim 1 or 2, **characterised in that** either one of the supports (1) with associated information molecules and sample (8) is added to the fluid either before, after or simultaneously as the fluid is placed on the substrate (49), **in that** step (c) is performed either before, after or simultaneously with step (b), and that step (f) is performed either before or after step (d).

4. A method according to Claim 1, 2 or 3, **characterised in that** the method further includes a step of using a measuring unit (35) for detecting the interaction signals and for reading the bar codes (2), the measuring unit (35) being arranged to detect the interaction signals and for reading of the bar codes (2) substantially simultaneously, the measuring unit (35) comprising the detecting and reading means (37, 40) for interrogating the supports (1).

5. A method according to any one or more of Claims 1 to 4, **characterised in that** signal emitting labels (9) are added to the fluid, said emitting labels (9) arranged to emit the interaction signals when information molecules (7) bind to molecules in the sample exhibiting one or more genetic characteristics.

6. A method according to any one or more of Claims 1 to 5, **characterised in that** the method further comprises a step of reading the fluid along a predetermined path (60) along the substrate (49) using a measuring unit (35) arranged for detecting and reading the supports (1), said path (60) arranged to receive substantially all of the fluid.

7. A method to any one or more of Claims 1 to 6, **characterised in that** the method further comprises a step of interrogating individual supports (1) only once.

8. A method according to any one or more of Claims 1 to 5, **characterised in that** the fluid, said fluid comprising the supports (1) with associated information molecules (7) and the sample including molecules susceptible to exhibiting at least one potential genetic characteristic (8), is passed through an interrogation zone (34) of a measuring unit (35) via focusing means for aligning and separating the supports (1) prior to interrogation.

9. A method according to any one or more of Claims 1 to 8, **characterised in that** a measuring unit (35) verifies reading and detection information from the supports (1) with a database including preset information linking specific bar codes (2) to specific information molecules (7).

10. A method according to any one of Claims 1 to 9, **characterised in that** the supports (1) are coated with a binding promoter on one or more of their main surfaces (11) to facilitate molecule attachment thereto.

11. A method according to Claim 10, **characterised in that** the binding promoter is at least one or silane and avidin-biotin.

12. A method according to any one of more of Claims 1 to 11, **characterised in that** the fluid includes a liquid solution.

13. A method according to any one or more of Claims 1 to 12, **characterised in that** one or more genetic characteristics being detected is gene expression analysis and the specific types of information molecules (7) being attached to the supports (1) are nucleic acid and/or peptide nucleic acid molecules.

14. A method according to any one or more of Claims 1 to 12, **characterised in that** one or more genetic characteristics being detected is Single Nucleotide Polymorpohisms (SNPs) detection/scoring and the specific types of information molecules (7) being attached to the supports (1) are nucleic acid and/or peptide nucleic acid molecules.

15. A method according to any one or more of Claims 1 to 12, **characterised in that** one or more genetic characteristics being detected is nucleic acid testing and the specific types of information molecules (7) being attached to the supports (1) are nucleic acid and/or PNA information molecules.

16. A method according to any one or more of Claims 1 to 12, **characterised in that** one or more genetic characteristics being detected is for drug target association testing and the specific types of information molecules (7) being attached to the supports (1) are nucleic acid and/or PNA information molecules.

17. A method according to any one or more of Claims 1 to 12, **characterised in that** one or more genetic characteristics being detected is for pharmacogenomic testing and the specific types of information molecules (7) being attached to the supports (1) are nucleic acid and/or PNA information molecules.

## Patentansprüche

1. Biochemisches Verfahren zur Detektion eines oder mehrerer genetischer Merkmale, worin das Verfahren Träger (1) verwendet, deren größte Dimension (3) geringer als 250 µm ist, und worin jeder Träger (1) sequenzielle Identifikationsmittel (2) aufnimmt, wobei das Verfahren folgende Schritte umfasst:
(a) Anheften eines Informationsmoleküls (7) an die Hauptoberfläche (11) jedes Trägers (1);
(b) Suspendieren der Träger (1) mit dem assoziierten Informationsmolekül (7) in einem Fluid;
(c) Zugeben einer zu analysierenden Probe (8) zum Fluid;
(d) Positionieren des Fluids, das assoziierte Träger (1) und eine Probe (8) umfasst, auf ein Substrat (49) zum darauf folgenden Abfragen;
(e) Detektion von Wechselwirkungssignalen von zumindest einem der Träger (1) im Fluid mithilfe von Signaldetektionsmittel (40); und
(f) Ablesen der sequenziellen Identifikationsmittel (2) der Träger (1), die ein Wechselwirkungssignal aufweisen, unter Verwendung von Lesemittel (3), wodurch zumindest ein oder mehrere genetische(s) Merkmal(e) (8) detektiert wird/werden,
**dadurch gekennzeichnet, dass**
(g) das Informationsmolekül (7) mit zumindest einem oder mehreren genetischen Merkmalen wechselwirken kann; und
(h) das sequenzielle Identifikationsmittel (2) ein Strichcode ist und zumindest eine der räumlichen Orientierungsmerkmale und Fehlerkorrekturmerkmale zur Unterstützung der Lesemittel (3) zur Bestimmung der Identitäten der Träger (1) vollständig aufnimmt.

2. Verfahren nach Anspruch 1, **dadurch gekennzeichnet, dass** das Verfahren weiters einen Schritt zur Oxidation der Träger (1) vor Anheftung der assoziierten Informationsmoleküle (7) umfasst.

3. Verfahren nach Anspruch 1 oder 2, **dadurch gekennzeichnet, dass** einer der Träger (1) mit assoziierten Informationsmolekülen und Probe (8) zu dem Fluid entweder bevor, nachdem oder zur selben Zeit hinzugefügt wird, wie das Fluid auf das Substrat (49) platziert wird, **dadurch**, dass Schritt (c) entweder vor, nach oder gleichzeitig mit Schritt (b) durchgeführt wird, und **dadurch**, dass Schritt (f) vor oder nach Schritt (d) durchgeführt wird.

4. Verfahren nach Anspruch 1, 2 oder 3, **dadurch gekennzeichnet, dass** das Verfahren weiters einen Schritt zur Verwendung einer Messeinheit (35) zur Detektion der Wechselwirkungssignale und zum Ablesen der Strichcodes (2) umfasst, worin die Messeinheit (35) so angeordnet ist, um im Wesentlichen gleichzeitig die Wechselwirkungssignale zu detektieren und die Strichcodes (2) abzulesen, worin die Messeinheit (35) die Detektions- und Lesemittel (37, 40) zum Abfragen der Träger (1) umfasst.

5. Verfahren nach einem oder mehreren der Ansprüche 1 bis 4, **dadurch gekennzeichnet, dass** zum Fluid signalemittierende Markierungen (9) zugegeben werden, worin diese emittierenden Markierungen (9) so angeordnet sind, dass sie die Wechselwirkungssignale emittieren, wenn sich die Informationsmoleküle (7) an Moleküle in der Probe binden, die ein oder mehrere genetische Merkmale aufweisen.

6. Verfahren nach einem oder mehreren der Ansprüche 1 bis 5, **dadurch gekennzeichnet, dass** das Verfahren weiters einen Schritt zum Lesen des Fluids entlang eines vorgegebenen Wegs (60) entlang des Substrats (49) umfasst, worin eine Messeinheit (35) verwendet wird, die zur Detektion und zum Lesen der Träger (1) angeordnet, worin dieser Weg (60) so angeordnet ist, dass im Wesentlichen das gesamte Fluid empfangen wird.

7. Verfahren nach einem oder mehreren der Ansprüche 1 bis 6, **dadurch gekennzeichnet, dass** das Verfahren weiters einen Schritt des Abfragens bestimmter Träger (1) nur einmal umfasst.

8. Verfahren nach einem oder mehreren der Ansprüche 1 bis 5, **dadurch gekennzeichnet, dass** das Fluid, worin das Fluid die Träger (1) mit assoziierten Informationsmolekülen (7) und die Probe umfasst, welche Moleküle enthält, die zumindest ein potenzielles genetisches Merkmal (8) aufweisen können, mittels Fokussierungmittel zur Anordnung und Trennung von Trägern (1) vor dem Abfragen durch eine Abfragezone (34) einer Messeinheit (35) geleitet wird.

9. Verfahren nach einem oder mehreren der Ansprüche 1 bis 8, **dadurch gekennzeichnet, dass** eine Messeinheit (35) die Lese- und Detektionsinformation der Träger (1) mithilfe einer Datenbank mit voreingestellten Informationen verifiziert, die spezielle Strichcodes (2) mit speziellen Informationsmolekülen (7) verbindet.

10. Verfahren nach einem der Ansprüche 1 bis 9, **dadurch gekennzeichnet, dass** die Träger (1) mit einem Bindungspromotor auf einer oder auf mehreren ihrer Hauptoberflächen (11) beschichtet sind, um die Molekülanheftung daran zu erleichtern.

11. Verfahren nach Anspruch 10, **dadurch gekennzeichnet, dass** der Bindungspromotor zumindest einer von Silan oder Avidin-Biotin ist.

12. Verfahren nach einem oder mehreren der Ansprüche 1 bis 11, **dadurch gekennzeichnet, dass** das Fluid eine flüssige Lösung umfasst.

13. Verfahren nach einem oder mehreren der Ansprüche 1 bis 12, **dadurch gekennzeichnet, dass** eines oder mehrere der detektierten genetischen Merkmale Genexpressionsanalyse ist und die spezifischen Formen der Informationsmoleküle (7), die an den Trägern (1) angeheftet sind, Nucleinsäure- und/oder Peptidnucleinsäuremoleküle sind.

14. Verfahren nach einem oder mehreren der Ansprüche 1 bis 12, **dadurch gekennzeichnet, dass** eines oder mehrere der detektierten genetischen Merkmale die Detektion/Auswertung von Einzelnucleotidpolymorphismen (SNPs) ist und die spezifischen Formen jener Informationsmoleküle (7), die an den Trägern angeheftet sind, Nucleinsäure- und/oder Peptidnucleinsäuremoleküle sind.

15. Verfahren nach einem oder mehreren der Ansprüche 1 bis 12, **dadurch gekennzeichnet, dass** eines oder mehrere der detektierten genetischen Merkmale die Untersuchung von Nucleinsäure ist und die spezifischen Formen der Informationsmoleküle (7), die an den Trägern (1) angeheftet sind, Nucleinsäure- und/oder PNA-Informationsmoleküle sind.

16. Verfahren nach einem oder mehreren der Ansprüche 1 bis 12, **dadurch gekennzeichnet, dass** eines oder mehrere der detektierten genetischen Merkmale zur Untersuchung von Arzneimittel-Target-Assoziation dient/dienen und die spezifischen Formen der Informationsmoleküle (7), die an den Trägern (1) angeheftet sind, Nucleinsäure- und/oder PNA-Informationsmoleküle sind.

17. Verfahren nach einem oder mehreren der Ansprüche 1 bis 12, **dadurch gekennzeichnet, dass** eines oder mehrere der detektierten genetischen Merkmale zur pharmakogenomischen Untersuchung dient/dienen und die spezifischen Formen der Informationsmoleküle (7), die an den Trägern (1) angeheftet sind, Nucleinsäure- und/oder PNA-Informationsmoleküle sind.

## Revendications

1. Méthode biochimique pour détecter une ou plusieurs caractéristiques génétiques, la méthode utilisant des supports (1) dont la plus grande dimension (3) est inférieure à 250 µm et où chaque support (1) contient des moyens d'identification séquentielles (2), la méthode comprenant les étapes de:
(a) . attacher une molécule d'information (7) à une surface principale (11) de chaque support (1);
(b) . mettre les supports (1) avec la molécule d'information associée (7) en suspension dans un fluide;
(c) . ajouter un échantillon (8) à analyser au fluide;
(d) . placer le fluide comprenant des supports associés (1) et l'échantillon (8) sur un substrat (49) pour une interrogation subséquente;
(e) . détecter les signaux d'interaction d'au moins l'un des supports (1) dans le fluide en utilisant un moyen de détection de signaux (40); et
(f) . lire le moyen d'identification séquentiel (2) des supports (1) qui ont un signal d'interaction en utilisant un moyen de lecture (3) pour ainsi détecter au moins l'une desdites une ou plusieurs caractéristiques génétiques;
**caractérisée en ce que**
(g) . la molécule d'information (7) est capable d'interagir avec au moins l'une desdites une ou plusieurs caractéristiques génétiques;
(h) . le moyen d'identification séquentiel (2) est un code barre et incorpore au moins l'un des traits d'orientation spatiale et des traits de correction d'erreurs pour aider le moyen de lecture (3) à déterminer les identités des supports (1).

2. Méthode selon la revendication 1, **caractérisée en ce que** la méthode comprend de plus une étape d'oxydation des supports (1) avant attachement des molécules associées d'information (7).

3. Méthode selon la revendication 1 ou 2, **caractérisée en ce que** l'un des supports (1) avec les molécules associées d'information et l'échantillon (8) est ajouté au fluide soit avant, après ou simultanément tandis que le fluide est placé sur le substrat (49), **en ce que** l'étape (c) est accomplie soit avant, après ou simultanément avec l'étape (b) et **en ce que** l'étape (f) est accomplie soit avant ou après l'étape (d).

4. Méthode selon la revendication 1, 2 ou 3, **caractérisée en ce que** la méthode comprend de plus une étape d'utilisation d'une unité de mesure (35) pour détecter les signaux d'interaction et pour lire les codes barre (2), l'unité de mesure (35) étant agencée pour détecter les signaux d'interaction et pour lire les codes barre (2) sensiblement simultanément, l'unité de mesure (35) comprenant les moyens de détection et de lecture (37, 40) pour interroger les supports (1).

5. Méthode selon une ou plusieurs des revendications 1 à 4, **caractérisée en ce que** des marqueurs émettant des signaux (9) sont ajoutés au fluide, lesdits marqueurs émetteurs (9) agencés pour émettre les signaux d'interaction quand les molécules d'information (7) se lient aux molécules dans l'échantillon présentant une ou plusieurs caractéristiques génétiques.

6. Méthode selon une ou plusieurs des revendications 1 à 5, **caractérisée en ce que** la méthode comprend de plus une étape de lecture du fluide le long d'un trajet prédéterminé (60) le long du substrat (49) en utilisant une unité de mesure (35) agencée pour détecter et lier les supports (1), ledit trajet (60) étant agencé pour recevoir sensiblement la totalité du fluide.

7. Méthode selon l'une quelconque des revendications 1 à 6, **caractérisée en ce que** la méthode comprend de plus une étape d'interrogation des supports individuels (1) une fois seulement.

8. Méthode selon une ou plusieurs des revendications 1 à 5, **caractérisée en ce que** le fluide, ledit fluide comprenant les supports (1) avec des molécules associées d'information (7) et l'échantillon contenant des molécules susceptibles de présenter au moins une caractéristique génétique potentielle (8), passe à travers une zone d'interrogation (34) d'une unité de mesure (35) via un moyen de focalisation pour aligner et séparer les supports (1) avant interrogation.

9. Méthode selon une ou plusieurs des revendications 1 à 8, **caractérisée en ce qu'**une unité de mesure (35) vérifie la lecture et la détection de l'information des supports (1) au moyen d'une base de données comprenant une information préétablie enchaînant des codes barre spécifiques (2) à des molécules spécifiques d'information (7).

10. Méthode selon l'une quelconque des revendications 1 à 9, **caractérisée en ce que** les supports (1) sont enduits d'un promoteur de liaison sur une ou plusieurs de leurs surfaces principales (11) pour faciliter l'attachement des molécules.

11. Méthode selon la revendication 10, **caractérisée en ce que** le promoteur de liaison est au moins l'un de silane et avidine-biotine.

12. Méthode selon une ou plusieurs des revendications 1 à 11, **caractérisée en ce que** le fluide contient une solution liquide.

13. Méthode selon une ou plusieurs des revendications 1 à 12, **caractérisée en ce qu'**une ou plusieurs caractéristiques génétiques détectées est l'analyse de l'expression d'un gène et les types spécifiques de molécules d'information (7) qui sont attachées aux supports (1) sont des molécules d'acide nucléique et/ou d'acide nucléique peptidique.

14. Méthode selon une ou plusieurs des revendications 1 à 12, **caractérisée en ce qu'**une ou plusieurs caractéristiques génétiques qui sont détectées est des Polymorpohismes de Nucléotides Simple (SNP), la détection/notation et les types spécifiques de molécules d'information (7) qui sont attachées aux supports (1) sont des molécules d'acide nucléique et/ou d'acide nucléique peptidique.

15. Méthode selon une ou plusieurs des revendications 1 à 12, **caractérisée en ce qu'**une ou plusieurs caractéristiques génétiques détectées est un test d'acide nucléique et les types spécifiques de molécules d'information (7) qui sont attachées aux supports (1) sont des molécules d'information d'acide nucléique et/ou PNA.

16. Méthode selon une ou plusieurs des revendications 1 à 12, **caractérisée en ce qu'**une ou plusieurs caractéristiques génétiques détectées est pour des tests d'association de cibles de produits pharmaceutiques et les types spécifiques de molécules d'information (7) qui sont attachées aux supports (1) sont des molécules d'information d'acide nucléique et/ou de PNA.

17. Méthode selon une ou plusieurs des revendications 1 à 12, **caractérisée en ce qu'**une ou plusieurs caractéristiques génétiques détectées est pour un test pharmacogénomique et les types spécifiques de molécules d'information (7) attachées aux supports (1) sont des molécules d'information d'acide nucléique et/ou de PNA.
